# EUROPÄISCHE PATENTSCHRIFT

(11) **EP 0 472 132 B1**
(45) Veröffentlichungstag und Bekanntmachung des Hinweises auf die Patenterteilung: **28.12.1994**
(21) Anmeldenummer: 91113829.5
(22) Anmeldetag: 18.08.1991
(51) Int. Cl.: A61B 17/54, A45D 29/04

(54) **Vorrichtung zur abtragenden Behandlung von Hornhaut**
Device for abrasive treatment of calloused skin
Dispositif pour le traitement abrasif des durillons

(30) Priorität: 22.08.1990 DE 9012066 U
(43) Veröffentlichungstag der Anmeldung: 26.02.1992
(73) Patentinhaber: CREDO STAHLWARENFABRIK GUSTAV KRACHT, D-42653 Solingen (DE)
(72) Erfinder: Gilhaus, Heinz, W-5650 Solingen 11 (DE)
(74) Vertreter: Plöger, Ulrich

(56) Entgegenhaltungen:
- DE-U- 1 985 681
- US-A- 2 663 070
- US-A- 4 537 207

## Beschreibung

Vorrichtung zur abtragenden Behandlung von Hornhaut, mit einem Handgriff und einer daran angesetzten Kopfplatte, die wahlweise eine Hobelklinge mit einer Deckplatte oder eine Raspelplatte tragen kann, wobei die Deckplatte bzw. die Raspelplatte im Bereich ihrer zur Grifflängsrichtung parallelen Ränder der Kopfplatte derart abgekantet sind, daß die Ränder die entsprechenden Kopfplattenränder haltend umgreifen.

Eine Vorrichtung dieser Art ist nach dem DE-U-19 85 681 bekannt. Dabei wird ein Hornhauthobel bedarfsweise auch als Hornhautraspel verwendbar gestaltet, indem ein Hornhauthobel mit einer der Wölbung der Kopfplatte angepassten Raspelplatte mit am Rand hochgebogenen, federnden Randteil ausgestattet wird, so daß die federnden Randteile die Raspelplatte auf die Kopfplatte aufzuschieben gestatten. Während die Lage der Hobelklinge durch eine entsprechende Gestaltung der Kopfplatte fixiert ist, so daß sie bei der Benutzung des Gerätes und den dabei in der Grifflängsrichtung wirksam werdenden Kräften eine Verschiebung erfahren kann, ist die Raspelplatte umso leichter aus ihrer Lage zu verschieben, je stärker die in der Grifflängsrichtung wirksamen Kräfte werden. Die bekannte Vorrichtung trägt somit zwar den bei dem Hornhauthobel auftretenden Beanspruchungen Rechnung, nicht jedoch denjenigen Belastungen, die sich bei einem Hornhautraspel ergeben, wenn dieser in der hauptsächlichen Arbeitsrichtung betätigt wird.

Von diesem Stand der Technik ausgehend liegt der Erfindung die Aufgabe zugrunde, eine Vorrichtung zur abtragenden Hornhautbehandlung derart zu gestalten, daß sie nicht nur als Hornhauthobel funktioniert, sondern darüberhinaus auch die an eine Hornhautraspel gestellten Anforderungen erfüllt, wie sie sich jedenfalls dann ergeben, wenn ein Hornhauthobel in der Grifflängsrichtung wirkend benutzt wird.

Zur Lösung dieser Aufgabenstellung schlägt die Erfindung vor, daß der nicht abgekantete Bereich der Raspelplatte in Form und Größe mit dem Äusseren der Kopfplatte übereinstimmt, und daß, in Grifflängsrichtung gesehen, vor und hinter den abgekanteten Rändern der Raspelplatte zur Grifflängsrichtung nicht parallele Randteile der Raspelplatte etwa rechtwinklig zur Kopfplatte hin abgekantet sind, wobei die Randteile etwa so breit sind wie die Kopfplatte dick ist.

Man kommt auf diese Weise zu einer Vorrichtung, bei welcher die Raspelseite nicht mehr durch ein Aufschieben, sondern lediglich durch ein Aufklippen mit der Kopfplatte zu verbinden ist. Bei einem Aufschieben würden die zur Kopfplatte hin abgekanteten Randteile entgegenstehen, die nicht zur Grifflängsrichtung parallel verlaufen. Diese hochgekanteten Bereiche bilden mit den übrigen abgekanteten Bereichen eine Art Rahmen für die Kopfplatte, der eine Fixierung in jeglicher Richtung ermöglicht. Bei der Kopfplatte wird von einer üblichen Form ausgegangen, wie sie zum Stand der Technik zählt, wobei es, vom Handgriff ausgesehen, im Anschluß an einen schmalen Übergangsabschnitt zunächst zu einer Verbreiterung der Kopfplatte kommt, die sich bis zu einem vorderen, quer verlaufenden Steg erstreckt. Die konstruktive Umgestaltung der Raspelplatte führt hier also zu einer beim Stand der Technik nicht vorgesehenen Wirkung, die zunächst darin besteht, daß die Raspelplatte nicht aufgeschoben, sondern aufgeklippt wird, und die insbesondere darin zu sehen ist, daß sich infolge der randseitigen Abstützung der Raspelplatte an der Kopfplatte keine Verschiebemöglichkeiten bei der normalen Benutzung mehr ergeben.

Die neue Raspelplatte gestattet insbesondere auch die bewährte Ausbildung einer Wölbung bei der Kopfplatte, in deren Richtung auch die Raspelplatte gekrümmt ist, jedoch mit einem größeren Krümmungsradius. In Verbindung mit einer leichten Federung führt dies dazu, daß die Verbindung mit der Kopfplatte form- und kraftschlüssig ist, da die Raspelplatte durch das Aufklippen auf der Kopfplatte eine leichte, elastische Biegung erfährt. Sie liegt in diesem Zustand flächig an den Stützflächen der Kopfplatte an.

Sofern die Raspelplatte stärker gekrümmt wird, erreicht man eine Hohllage, der dadurch entsprochen werden muß , daß die Raspelplatte entsprechend breit ist, und daß die zusätzlich abzukantenden Randteile hinreichend breit sind, um an den zur Grifflängsrichtung nicht parallelen Außenkanten der Kopfplatte zur Anlage zu kommen.

Für die Anwendung hat sich in der Praxis gut bewährt, die parallel zur Grifflängsrichtung abgekanteten Ränder breiter zu wählen, als dies für die Umgreifung der Kopfplattenränder zwingend notwendig wäre. Durch eine entsprechende Verbreiterung lassen sich die Ränder zunächst nach innen biegen, so daß sie die Kopfplattenränder zuverlässig zu umgreifen vermögen. Sodann können sie abschließend noch mit einem Ausmaß hochgebogen werden, welches für ein Aufklippen eine Anlauffläche der Kopfplattenrandkanten und für ein Abnehmen eine Fingeranlage bildet. Somit läßt sich nicht nur neuerungsgemäß eine verbesserte Festlegung der Raspelplatte in Bezug auf die Kopfplatte erreichen, sondern auch die unter dem Gesichtspunkt des Austausches notwendigen Handhabungen sind erleichtert.

Zur weiteren Veranschaulichung der Erfindung wird auf die sich auf Ausführungsbeispiele beziehenden Zeichnungen Bezug genommen. Darin zeigen:
- Figur 1:: Den bekannten Hornhauthobel mit Klinge und Deckplatte,
- Figur 2:: eine einzelne Klinge,
- Figur 3:: die Deckplatte,
- Figur 4:: den Hornhauthobel ohne Klinge und Deckplatte,
- Figur 5:: den erfindungsgemäß ausgeführten Hornhauthobel als Hornhautraspel, mit Blick auf die Rückseite der Kopfplatte,
- Figur 6:: eine entsprechende Ausführung gemäß Figur 5 In vorderseitiger Ansicht,
- Figur 7:: eine entsprechende Ausführungsform nach Figur 5 in seitlicher Ansicht,
- Figur 8:: die neue Raspelplatte in Schrägansicht ihrer Rückseite,
- Figur 9:: die neue Raspelplatte in Draufsicht auf ihre Rückseite und
- Figur 10:: die neue Raspelplatte in Vorderansicht.

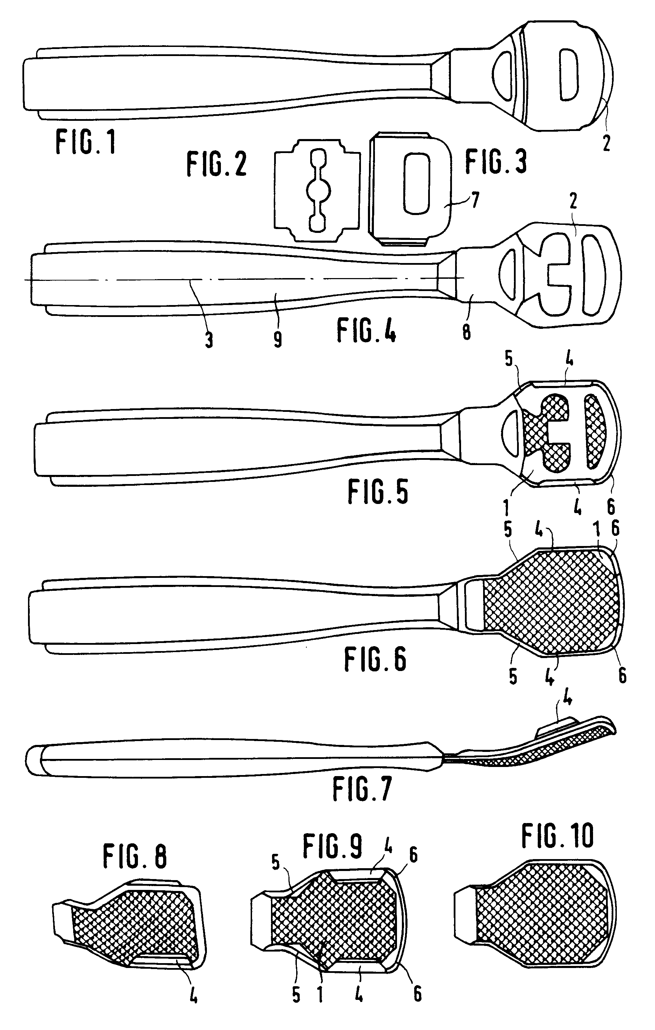

Figur 1 zeigt den grundsätzlich bekannt gewesenen Hornhauthobel, wie er der vorliegenden Erfindung als Stand der Technik zugrunde liegt. Die Klinge gemäß Figur 2 sowie die in Figur 3 wiedergegebene Deckplatte 7 sind auf die Kopfplatte 2 aufgesetzt.

Figur 4 zeigt die Hornhauthobel entsprechend Figur 1 noch ohne Klinge und Deckplatte. Die Kopfplatte 2 laßt entsprechend Montageöffnungen erkennen, die in bekannter Weise zur Anordnung der Klinge und der Deckplatte dienen. Die Kopfplatte 2 geht mit einen schmalen Übergangsstück 8 in den Handgriff 9 über. Die Grifflängsrichtung 3 veranschaulicht zugleich die Richtung, in welcher bei Benutzung des Gerätes größere Kräfte auftreten. Im übrigen entspricht Figur 4 den Stand der Technik, von dem die Neuerung ausgeht.

In der rückseitigen Ansicht, in der sich die Raspelplatte 1 im Hinblick auf die Kopfplatte 2 aufgesetzten Zustand gemäß Figur 5 zeigt, sind vor allem die abgekanteten Ränder 4 parallel zur Grifflängsrichtung 3 erkennbar. Die weiteren abgekanteten Randteile 5 und 6 verlaufen nicht mehr parallel zur Grifflängsrichtung 3 und vermögen auf Grund dieser Lage die erfindungsgemäße Sperrwirkung zu erzielen.

Die Arbeitsfläche der Raspelplatte 1 erkennt man in Figur 6. Von den abgekanteten Rändern 4 erkennt man lediglich die Kantenlinien. Die Greifmöglichkeit der abgekanteten Ränder 4 wird vor allem aus Figur 7 deutlich. Einerseits unterstützen die verbreiterten Ränder in der vorgeschlagenen Abkantung das Aufklippen, indem sie eine Gleitfläche für das Auflaufen der Kopfplattenkanten bilden. Andererseits wird hierdurch das gerade für die vorliegende Erfindung so wesentliche Wechseln vom Raspel zum Hobel und umgekehrt erleichtert, Die einzelnen Abkantbereiche 4,5 und 6 sind in den lediglich die Raspelplatten wiedergebenden Figuren 8 und 9 nochmals ersichtlich dargestellt. Wie vor allem Figur 9 erkennen läßt, schließt die Raspelplatte 1 die Kopfplatte 2 in aufgesetztem Zustand praktisch völlig in sich ein. Dabei läßt sich die Arbeitsfläche entsprechend Figur 10 bis weit in den Übergangsabschnitt 8, wie aus Figur 4 hervorgeht, erstrecken.

## Patentansprüche

1. Vorrichtung zur abtragenden Behandlung von Hornhaut, mit einem Handgriff (9) und einer daran angesetzten Kopfplatte (2), die wahlweise eine Hobelklinge mit einer Deckplatte (7) oder eine Raspelplatte (1) tragen kann, wobei die Deckplatte (7) bzw. die Raspelplatte (1) im Bereich ihrer zur Grifflängsrichtung (3) parallelen Ränder (4) der Kopfplatte (2) derart abgekantet sind, daß die Ränder (4) die entsprechenden Kopfplattenränder haltend umgreifen,
dadurch gekennzeichnet,
daß der nicht abgekantete Bereich der Raspelplatte (1) in Form und Größe mit dem Äusseren der Kopfplatte (2) übereinstimmt, und daß, in Grifflängsrichtung (3) gesehen, vor und hinter den abgekanteten Rändern (4) der Raspelplatte (1) zur Grifflängsrichtung (3) nicht parallele Randteile (5, 6) der Raspelplatte (1) etwa rechtwinklig zur Kopfplatte (2) hin abgekantet sind, wobei die Randteile (5, 6) etwa so breit sind wie die Kopfplatte (2) dick ist.

2. Vorrichtung nach Anspruch 1,
dadurch gekennzeichnet,
daß die Kopfplatte (2) und die Raspelplatte (1) sowie auch die Deckplatte (7) in gleicher Richtung gewölbt ausgeführt sind, wobei die Raspelplatte (1) einen größeren Krümmungsradius als die Kopfplatte (2) besitzt und leicht federnd ist.

3. Vorrichtung nach Anspruch 1,
dadurch gekennzeichnet,
daß die Kopfplatte (2) und die Raspelplatte (1) sowie auch die Deckplatte (7) in gleicher Richtung gewölbt ausgeführt sind, wobei die Raspelplatte (1) derart gekrümmt ist, daß sie für ein Aufklippen auf der Kopfplatte (2) mit ihren zur Grifflängsrichtung (3) parallelen Rändern (4) zu spreizen ist.

4. Vorrichtung nach den Ansprüchen 1 bis 3,
dadurch gekennzeichnet,
daß die parallel zur Grifflängsrichtung (3) abgekanteten Ränder (4) der Raspelplatte (1) über die entsprechenden, von ihnen umgriffenen Kopfplattenränder hinaus zunächst nach innen gebogen und sodann abschließend mit einem Maß hochgebogen wird, welches für ein Aufklippen eine Anlauffläche der Kopfplattenrandkante und für ein Abnehmen eine Fingeranlage bildet.

## Claims

1. Device for the abrasive treatment of calluses, with, a hand grip (9) and a head plate (2) attached thereto that may, as required, carry a razor blade with a cover plate (7) or a rasp plate (1), the cover plate (7) or the rasp plate (1) comprising edges (4) that are parallel to the hand grip's longitudinal axis (3) and are folded towards the head plate (2) such that the edges (4) of the cover plate (7) or of the rasp plate (1) slip over and hold the corresponding edges on the head plate,
characterized in that
the non-folded area of the rasp plate (1) corresponds in shape and size with the outside of the head plate (2), and that, with reference to the grip's longitudinal axis (3) and on either side of the folded edges (4) of the rasp plate (1), non parallel edge sections (5, 6) of the rasp plate (1) are folded at about 90° towards the head plate (2), whereby the edge sections (5, 6) are approximately as broad as the head plate (2) is thick.

2. Device according to Claim 1,
characterized in that
the head plate (2) and the rasp plate (1) as well as the cover plate (7) are crowned in the same direction, whereby the rasp plate (1) comprises a larger crowing radius than the head plate (2) and is slightly resilient.

3. Device according to Claim 1,
characterized in that
the head plate (2) and the rasp plate (1) as well as the cover plate (7) are crowned in the same direction, whereby the rasp plate (1) is crowned such that it may be expanded to allow it to be clipped on to the head plate (2) by means of its edges (4) parallel to the grip's longitudinal axis (3).

4. Device according to Claims 1 to 3,
characterized in that
the folded edges (4) of the rasp plate (1) that are parallel to the grip's longitudinal axis (3) are first bent inwards over the head plate edges that they enclose and then bent upwards to a certain extent, thus forming a stopping face for the head plate edges during clipping on and a finger rest for removal.

## Revendications

1. Dispositif de traitement de durillons par enlèvement présentant une poignée (9) et une plaque supérieure (2) y faisant suite pouvant supporter, au choix, soit une lame de rabotage avec une plaque de recouvrement (7), soit une plaque à râper (1), la plaque de recouvrement (7) ou la plaque à râper (1) étant pliée dans le secteur des bords parallèles (4) à la direction longitudinale de la poignée (3) de la plaque supérieure (2). de telle sorte que les bords (4) recouvrent, en les maintenant, les bords correspondants de la plaque supérieure,
caractérisé en ce que
la forme et la taille du secteur non-plié de la plaque à râper (1) correspondent à l'extérieur de la plaque supérieure (2), et que devant et derrière les bords pliés (4) de la plaque à râper (1) vu dans la direction longitudinale de la poignée (3), des parties (5, 6) de bordure non parallèles à la direction longitudinale de la poignée (3) de la plaque à râper (1) sont recourbés pratiquement perpendiculairement à la plaque supérieure (2), les parties de bordure (5, 6) présentant une largeur pratiquement égale à l'épaisseur de la plaque supérieure (2).

2. Dispositif selon la revendication 1,
caractérisé en ce que
la plaque supérieure (2) et la plaque à râper (1), tout comme la plaque de recouvrement (7) sont réalisées avec une courbure allant dans la même direction, la plaque à râper (1) présentant un rayon de courbure supérieur à celui de la plaque supérieure et étant légèrement élastique.

3. Dispositif selon la revendication 1,
caractérisé en ce que
la plaque supérieure (2) et la plaque à râper (1), tout comme la plaque de recouvrement (7) sont réalisées avec une courbure allant dans la même direction, la plaque à râper (1) étant recourbée de telle sorte que ses bords parallèles (3) à la direction longitudinale de la poignée (4) peuvent être écartés pour une fixation par encliquetage.

4. Dispositif selon les revendications 1 à 3, caractérisé en ce que les bords pliés (4) parallèlement à la direction longitudinale de la poignée (3) de la plaque à râper (1) sont tout d'abord repliés vers l'intérieur au-dessus des bords de la plaque supérieure et sont ensuite repliés vers le haut d'une dimension formant une surface de départ du bord de la plaque frontale pour un encliquetage et une surface de pose du doigt pour le retrait.
